# FASCICULE DE BREVET EUROPEEN

(11) **EP 3 016 927 B1**
(45) Date de publication et mention de la délivrance du brevet: **03.01.2018**
(21) Numéro de dépôt: 14739890.3
(22) Date de dépôt: 25.06.2014
(51) Int. Cl.: C07C 45/54, C07C 49/653

(54) **NOUVEAU PROCÉDÉ DE SYNTHÈSE DE LA KHUSIMONE**
NEUARTIGES VERFAHREN ZUR SYNTHESE VON KHUSIMON
NOVEL METHOD FOR THE SYNTHESIS OF KHUSIMONE

(30) Priorité: 03.07.2013 FR 1356478
(43) Date de publication de la demande: 11.05.2016
(73) Titulaire: Robertet S.A., 06130 Grasse (FR); Université de Nice Sophia Antipolis Dirved, 06103 Nice Cedex 2 (FR); Centre National de la Recherche Scientifique (C.N.R.S.), 75794 Paris Cedex 16 (FR)
(72) Inventeur: BELHASSEN, Emilie, F-06700 Saint-Laurent-du-Var (FR); BALDOVINI, Nicolas, F-06200 Nice (FR); FILIPPI, Jean-Jacques, 06100 Nice (FR); BREVARD, Hugues, F-06130 Grasse (FR)
(74) Mandataire: Santarelli
(86) Numéro de dépôt international: PCT/FR2014/051605
(87) Numéro de publication internationale: WO 2015/001225

(56) Documents cités:
- FR-A1- 2 201 841
- HSING-JANG LIU ET AL: "Total synthesis of zizaane sesquiterpenes: (-)-khusimone, (+)-zizanoic acid, and (-)-epizizanoic acid", CANADIAN JOURNAL OF CHEMISTRY, vol. 60, no. 9, 1 janvier 1982 (1982-01-01), pages 1081-1091, XP055098033, ISSN: 0008-4042, DOI: 10.1139/v82-161 cité dans la demande
- SAKURAI K ET AL.: "A Simple Preparation of (-)-Khusimone (1) Using Electrochemical Decarboxylative Acetylation", AGRICULTURAL AND BIOLOGICAL CHEMISTRY, vol. 53, no. 5, 23 mai 1989 (1989-05-23), pages 1449-1450, XP002719208, cité dans la demande
- JAIN S C ET AL: "Insect repellents from vetiver oil: I. zizanal and epizizanal", TETRAHEDRON LETTERS, PERGAMON, GB, vol. 23, no. 45, 1 janvier 1982 (1982-01-01), pages 4639-4642, XP026786928, ISSN: 0040-4039, DOI: 10.1016/S0040-4039(00)85675-0 [extrait le 1982-01-01]

## Description

Le terme "Vétiver" désigne en français des plantes de la famille des *Poaceae* (Graminées). Il s'agit de plusieurs espèces du genre *Chrysopogon* (anciennement *Vetiveria*)*.* On en connaît une douzaine d'espèces poussant dans les zones tropicales. La plus connue est *Chrysopogon zizanioides* qui pousse surtout sur le sous-continent indien. Deux autres espèces sont fréquemment cultivées : *Chrysopogon nigritanus* en Afrique australe et *Chrysopogon nemoralis* en Asie du Sud-Est.

La plante se présente sous forme de grandes touffes vertes, dont les racines, se développant verticalement et peuvent atteindre des profondeurs allant jusqu'à trois mètres.

Après distillation, les racines de vétiver fournissent une huile essentielle très visqueuse utilisée en parfumerie. C'est une essence à la saveur fine et complexe : boisée, aromatique, verte, terreuse, quelquefois légèrement fumée ou agrume.

De nombreux parfums sur le marché contiennent comme ingrédient clé de l'huile essentielle de vétiver ou ses dérivés comme par exemple les acétates de vétivéryle.

À l'heure actuelle, aucun matériau odorant synthétique correspondant à l'huile essentielle de vétiver n'est disponible dans le commerce.

L'absence de substitut synthétique est due en partie à la nature complexe des constituants de l'essence de vétiver.

Les études olfactométriques conduites par la demanderesse sur différents extraits, fractions, et dérivés de vétiver ont permis d'identifier plusieurs des principaux composés d'impact olfactif. Parmi ces molécules d'impact, celles présentant un squelette zizaane occupent une place particulière, notamment la khusimone et son isomère (par ailleurs dans le texte regroupés sous le terme khusimone) dont les structures correspondent à la formule I suivante 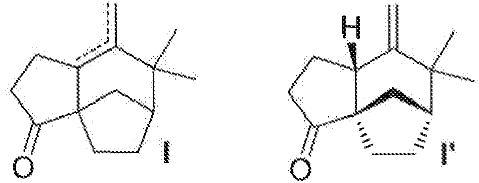 et particulièrement l'isomère de formule I' qui est reconnu comme un des principes odorants majeurs du vétiver. Cette substance présente une odeur boisée, typiquement vétiver et réminiscente de l'odeur de l'huile essentielle de vétiver.

Pour les raisons évoquées plus haut, la synthèse de la khusimone a rapidement suscité un grand intérêt, tant pour le challenge synthétique que représente la construction de son noyau tricyclique possédant plusieurs centres stéréogènes que pour ses potentialités d'utilisation dans l'industrie de la parfumerie. Un certain nombre de synthèses totales académiques ont été publiées, mais restent difficilement transposables à l'échelle industrielle.

En revanche, l'hémisynthèse de la khusimone à partir d'acide zizanoïque ou de sa forme ISO de formule II suivante 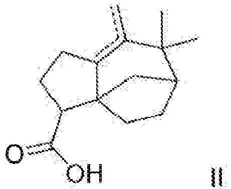 est bien plus prometteuse.

En effet, cet acide est naturellement présent, à des teneurs pouvant atteindre plus de 10%, dans certains extraits de vétiver, particulièrement dans les huiles essentielles, et peut en être isolé sélectivement par un simple lavage acido-basique.

Il est connu dans l'art antérieur trois méthodes permettant la conversion de l'acide zizanoïque en khusimone :
FR2 201 841 (3 mai 1974) décrit la transformation de l'acide zizanoïque en khusimone au travers d'une décarboxylation oxydative de l'acide zizanoïque en acétate de 12-norziza-6(13)-en-2β-yle à l'aide de tétracétate de plomb. Cet acétate peut être ensuite saponifié puis oxydé en khusimone. L'utilisation de 12 équivalents de tétracétate de plomb dans la première étape rend l'industrialisation de cette voie de synthèse impossible du fait de son coût et de son caractère polluant.

En 1980, Maurer B. (Seifen Öle Fette Wachse ; 106. Jg. 1980, 13, 347) propose une approche alternative dans laquelle le zizanoate de méthyle, traité par le tertiobutylate de potassium réagit avec l'oxygène pour conduire à un produit d'α-hydroxylation qui peut être ensuite dégradé en khusimone.

Hsing-Jang Liu et al. (Can. J. Chem., vol. 60, 1982) décrivent en 1982 la préparation d'acide zizanoïque par mise en présence du zizanal avec un réactif oxydant qui est le réactant de Jones, dont les résidus chromés sont très toxiques. En 1989, Sakurai et ses collaborateurs ont publié (Sakurai et coll., Agric. Biol. Chem., 1989, 53(5), 1449-1450) la conversion de l'acide zizanoïque en khusimone par voie électrochimique. Les mauvais rendements et le faible taux de conversion de cette voie de synthèse rendent cette dernière peu attractive pour son industrialisation.
Ainsi, il subsiste toujours un besoin en voies de synthèse de la khusimone facilement industrialisable parce que les réactifs utilisés sont économiques, écologiques et compatibles avec la santé publique, ou alors d'une voie de synthèse qui pourrait exonérer l'opérateur de l'emploi d'un catalyseur souvent coûteux et rendant généralement l'industrialisation de la réaction quasiment impossible.

C'est l'un des objets de la présente invention. En effet, les inventeurs ont trouvé une voie originale d'hémisynthèse de la khusimone. La nature des réactifs et des conditions employées permet d'envisager que cette nouvelle voie de synthèse soit adaptable à l'échelle industrielle et reste tout à fait compétitive.

Les inventeurs ont ainsi découvert que le zizanal de formule III 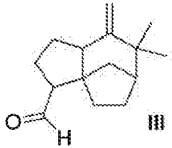 présente une réactivité inattendue et qu'il est possible de former un mélange de khusimone (I) et des alcools 12-norziza-6(13)-en-2α-ol et 12-norziza-6(13)-en-2β-ol (ou 12-norzizaenols) correspondants (IV) par décarbonylation oxydative dudit zizanal (III) selon le schéma réactionnel suivant :

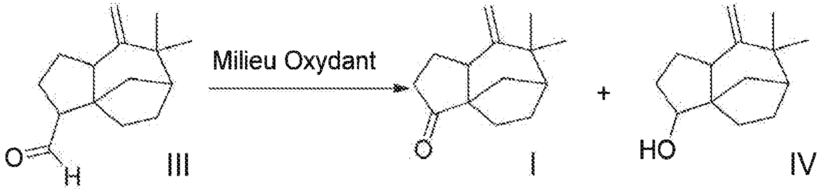

Du fait que les réactifs sont très économiques, non toxiques et non polluants, cette méthode d'hémisynthèse semble particulièrement adaptée à la production industrielle de la khusimone. De plus cette réaction peut s'effectuer en l'absence de tout catalyseur.Ainsi, l'invention a pour objet premier la synthèse de khusimone par décarbonylation du zizanal avec un réactif oxydant de type peracide ou peroxyde ou d'un mélange de peracides ou d'un mélange de peroxydes ou d'un mélange de peracides et de peroxydes eux-mêmes uniques ou un mélange. Selon une première variante, la réaction pourra être réalisée en présence d'une base.

Selon une autre variante, la réaction peut être réalisée en présence d'un solvant, préférentiellement un solvant organique.

Selon encore une autre variante, préférentielle, la réaction peut être réalisée en présence d'une base et d'un solvant organique.

Plus précisément, l'invention a pour objet la synthèse de khusimone par décarbonylation oxydative du zizanal comprenant la réaction du zizanal en présence d'un premier réactif oxydant de type peracide ou peroxyde ou d'un mélange, préférentiellement en présence d'une base ou d'un solvant, très préférentiellement en présence d'un base et d'un solvant, ledit solvant étant avantageusement un solvant organique.

Par mélange on entend mélange de peracides ou mélange de peroxydes ou mélange de peracides et de peroxydes qu'ils soient eux-mêmes uniques ou un mélange.

Selon l'invention, ledit réactif oxydant peut être choisi parmi l'eau oxygénée, l'hydroperoxyde de *tert*-butyle, le peroxyde de di-*tert*-butyle, le peroxyde de dicumyle, le peroxyde de *tert*-butyle et de cumyle, le peroxyde de dibenzoyle, de dilauryle, de di-(2.4-dichlorobenzoyle), l'hydroperoxyde de *tert*-butyle, de cumyle, de 1-phényléthyle, l'acide performique, l'acide peracétique, l'acide perpropionique, l'acide *m*-chloroperbenzoïque, l'acide monoperphtalique, l'acide monopermaléique ou encore l'acide trifluoroperacétique.

Avantageusement, ledit réactif oxydant est l'eau oxygénée.

Selon l'invention, la réaction de décarbonylation oxydative du zizanal peut être réalisée avec un rapport molaire [(RO)/(Z)] entre le réactif oxydant (RO) et le zizanal (Z) qui peut être compris entre 0,1 et 50, préférentiellement entre 0,5 et 25, très préférentiellement entre 1 et 10, avantageusement égal à 6.

Selon l'invention, la base peut être choisie parmi la soude (NaOH), la potasse (KOH), le méthanolate de sodium (MeONa), l'éthanolate de sodium (EtONa), le tertiobutylate de potassium (tBuOK), l'hydroxyde de calcium (Ca(OH)₂), l'ammoniaque, l'hydroxyde de lithium (LiOH).

Avantageusement ladite base peut être de la soude ou de la potasse, très avantageusement de la potasse.

Selon l'invention, la réaction de décarbonylation oxydative du zizanal peut être réalisée avec un rapport molaire [(B)/(Z)] entre la base (B) et le zizanal (Z) qui peut être compris entre 0,1 et 50, préférentiellement entre 0,5 et 25, très préférentiellement entre 1 et 10, avantageusement égale à 3.

Selon l'invention, ledit solvant peut être choisi parmi l'eau, le méthanol, l'éthanol, le propanol, l'isopropanol, le n-butanol, le *sec-*butanol, l'isobutanol, le *tert*-butanol, le têtrahydrofurane (THF), le dioxane, le méthyl *tert*-butyl éther (MTBE), l'éther diéthylique (Et₂O), le glyme, le diglyme, le dichlorométhane (CH₂Cl₂), le chloroforme (CHCl₃), le diméthylsulfoxyde (DMSO), l'acétonitrile, l'acétate d'éthyle, l'acétate d'isopropyle ou des mélanges de toute nature (binaire, ternaire, etc) et en toutes proportions de ces solvants comme par exemple les mélanges binaires DMSO/méthanol, THF/méthanol, DMSO/THF, DMSO/Dioxane.

Avantageusement ledit solvant organique peut être le méthanol ou un mélange MeOH/DMSO en toutes proportions, très avantageusement un mélange 1/1 MeOH/DMSO.

Selon l'invention, la réaction de décarbonylation oxydative du zizanal peut être réalisée à toutes les concentrations de zizanal dans le mélange réactionnel. Ainsi, selon l'invention, la réaction de décarbonylation oxydative du zizanal peut être réalisée dans une gamme de concentration en zizanal dans le mélange réactionnel comprise entre 0,01 et 4,5M, avantageusement entre 0,02 et 0,07M, très avantageusement 0,2M.

Il est également envisageable de ne pas utiliser de solvant du tout ou très peu. Ainsi selon une variante de l'invention, la réaction de décarbonylation oxydative du zizanal peut être réalisée sans solvant.

Selon l'invention, la réaction de décarbonylation oxydative du zizanal peut être réalisée à une température qui peut être comprise entre -25°C et la température de reflux du solvant. Avantageusement, la réaction peut être démarrée à une température comprise entre -25°C et 25°C, préférentiellement - 25°C et 0°C. La réaction peut ensuite évoluer librement jusqu'à la température de reflux du solvant et pourra ensuite être maintenue à une température comprise entre 25°C et le reflux, jusqu'à consommation des réactifs. Très préférentiellement, une fois atteinte la température de reflux du solvant, la réaction pourra être maintenue à cette température.

L'homme du métier saura sans difficulté arrêter la réaction quand il constatera par prélèvement et analyse que la réaction a atteint l'avancement désiré, par exemple, en constatant la consommation partielle ou totale du zizanal par analyse en chromatographie gazeuse ou chromatographie sur couche mince, ou encore par résonance magnétique nucléaire. Par exemple la réaction peut être arrêtée par ajout d'une solution aqueuse et d'un solvant organique où même une simple évaporation des solvants. La solution aqueuse peut être, par exemple : une solution de carbonate de calcium voir même une solution aqueuse réductrice, acide, neutre ou basique (comme par exemple une solution d'acide chlorydrique ou une solution de thiosulfate de sodium). Les solvants formant la phase organique peuvent être par exemple : l'acétate d'éthyle, l'éther diéthylique ou l'éther de pétrole, le MTBE, le cyclohexane, l'hexane, le pentane, le THF, voir même le dichlorométhane. Il est également possible de procéder à une filtration sur silice / celite, ou une simple évaporation des solvants.

A ce stade selon l'invention, le mélange obtenu après décarbonylation oxydative du zizanal et ajout de la solution d'arrêt peut être utilisé directement ou bien le mélange 12-norzizaenols (IV) / khusimone (I) peut être isolé du milieu réactionnel obtenu après décarbonylation oxydative du zizanal.

Selon l'invention, la réaction de décarbonylation oxydative du zizanal commence dès l'ajout des réactifs. La réaction peut être conduite pendant plusieurs jours sans subir de dégradation des produits.

Comme indiqué précédemment, la réaction de décarbonylation oxydative du zizanal conduit à l'obtention d'un mélange contenant la khusimone et les 12-norzizaenols. Les rendements et le ratio 12-norzizaenols (IV) / khusimone (I) en fin de réaction varient selon le type de base et le nombre d'équivalents des réactifs utilisés. On observe également que le ratio 12-norzizaenols (IV) / khusimone (I) varie selon le solvant utilisé. En effet, lorsque l'on utilise du méthanol seul, on observe majoritairement de la khusimone alors qu'un mélange DMSO/MeOH génère plus de 12-norzizaenols que de khusimone.

Toutes les méthodes connues de l'Homme du métier permettant d'isoler le mélange 12-norzizaenols (IV) / khusimone (I) peuvent être utilisées selon l'invention.

Une fois isolé, le mélange 12-norzizaenols (IV) / khusimone (I) peut être soumis à une oxydation en présence par exemple d'un oxydant (O) choisi parmi le perruthénate de tétrapropylammonium ou encore les hypochlorites alcalins tels que par exemple l'hypochlorite de calcium ou de sodium, l'oxyde de manganèse, le dichromate ou le chlorochromate de pyridinium, le nitrate de cérium et d'ammonium (CAN), l'oxydant de Collins, le trioxyde de chrome, le 2,3-dichloro-5,6-dicyano-*p*-benzoquinone (DDQ), la 2,4,6-trichloro-[1,3,5]-triazine, un mélange de diméthylsulfoxyde (DMSO) et de chlorure d'oxalyle (oxydation de Swern), un mélange de DMSO et du trioxyde de soufre pyridine (oxydation de Parikh-Doering), un mélange de DMSO et d'anhydride trifluoroacétique, un mélange de DMSO et d'anhydride acétique, un mélange de DMSO et d'oxyde de Phosphore (P₂O₅), un mélange de sulfure de diméthyle et de *N*-chlorosuccinimide (Me₂S/NCS oxydation de Corey-Kim), un mélange de H₂O₂ et de bicarbonate de potassium (KHCO₃) (oxydation de Fleming) le periodinane de Dess-Martin ou l'oxyde d'acétoxyiodine, l'acide 2-iodoxybenzoique, l'isopropoxyde d'aluminium, un système oxydant contenant le TEMPO (2,2,6,6-tetraméthylpiperidine-1-oxyl), ou le perruthénate de tetrapropylammonium (TPAP) et de la *N*-méthylmorpholine-*N-*oxyde, préférentiellement le perruthénate de tétrapropylammonium et éventuellement d'un co-oxydant tel que par exemple le N-oxyde de N-méthylmorpholine (NMO), en présence d'un solvant organique choisi parmi le dichlorométhane (DCM), l'éther diéthylique, le DMSO, le toluène, le tétrahydrofurane (THF), ou encore l'acétone, l'acétonitrile, l'eau, ou un mélange en toutes proportions des solvants cités, préférentiellement le DCM, l'acétone, le DMSO et l'eau ou un mélange en toutes proportions des solvants cités.

Cette oxydation a pour but de convertir les 12-norzizaenols (IV) en khusimone (I).

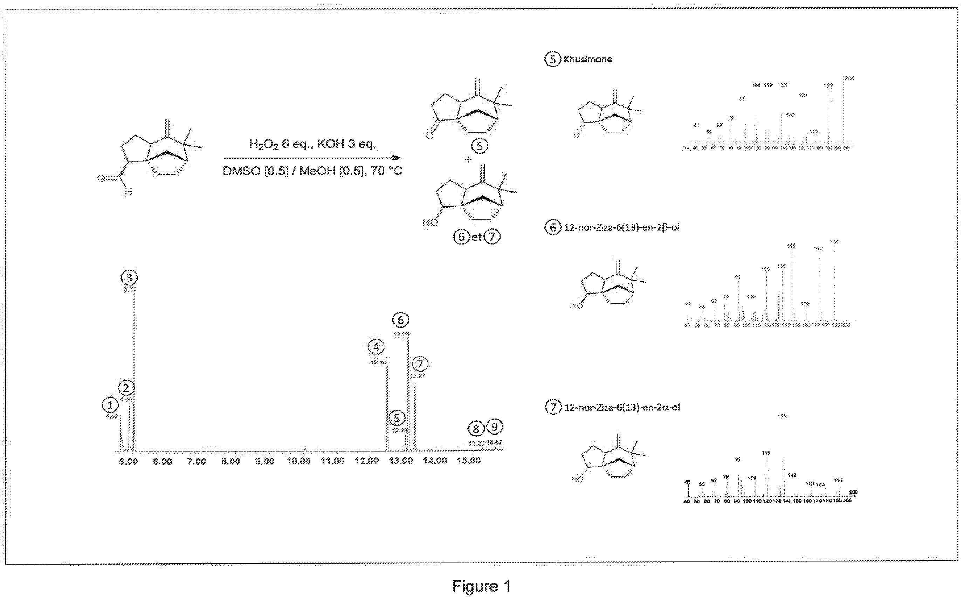

D'autres caractéristiques et avantages de l'invention ressortiront des exemples qui suivent, donnés à titre illustratif et non limitatif ainsi que de la figure 1 annexée qui présente les résultats de l'analyse par chromatographie en phase gazeuse de la réaction de décarbonylation oxydative du zizanal en présence d'eau oxygénée.

### Exemples :

### Exemple 1 : Synthèse de la khusimone à partir de zizanal en présence d'eau oxygénée (H₂O₂) :

Dans un ballon, 1 équivalent de zizanal est mis en présence de 6 équivalents d'eau oxygénée (35% p/p dans l'eau) et de 3 équivalents de potasse (6N dans l'eau) à une concentration finale de 0,2 mol/L de zizanal dans le mélange réaction qui contient en outre un mélange 1/1 de MeOH/DMSO, au reflux (76 °C). pendant 4h.
L'avancement de la réaction peut être suivi par l'analyse de prélèvements du milieu réactionnel. Une fois la conversion souhaitée atteinte, la réaction est traitée par addition d'une solution saturée de bicarbonate de sodium (NaHCO₃) dans le milieu réactionnel. On ajoute ensuite une quantité équivalente d'éther diéthylique. La phase organique est récupérée. La phase aqueuse est mise en présence d'acide chlorhydrique (HCl) 2M pour arriver à pH acide (quasiment 0) puis ladite phase aqueuse acidifiée est réextraite 3 fois avec de l'éther diéthylique. Une fois réunies, les phases organiques sont lavées à la saumure puis séchées avec du sulfate de magnésium et enfin filtrées pour être évaporées.

Le résultat de cette réaction est présenté à la figure 1 dans laquelle on peut voir le chromatogramme de suivi de cette réaction. On observe la disparition totale du composé de départ (Zizanal) et la présence de 12-norzizaenol [pics (6) et (7)] et de khusimone [pic (5)] qui sont quantifiés par deux références internes, l'anisole (pic 3) et l'hexadécane (pic 4). Le chromatogramme fait apparaître un pic d'un produit inconnu (1), in pic correspondant à la diméthylsulfone (pic 2), ainsi que 2 pics [(8 et (9)] correspondants respectivement au diméthylacétal de zizanyle (pic 8) et à l'acide zizanoïque (pic 9).

Ces résultats ont aussi été confirmés par purification des produits sur colonne de silice.

Le mélange obtenu est composé de 6% de khusimone, de 75% de 12-norzizaenols, et de 1 % de zizanal dimethylacétal

### Exemple 2 : Synthèse de la khusimone à partir du mélange khusimone/zizaenol obtenu à l'exemple 1

Dans un ballon, 1 équivalent du mélange zizaenol/khusimone obtenu à l'exemple 1 est mis en présence de 0,01 équivalent de perruthénate de tétrapropylammonium (TPAP) et de 0,7 équivalents de N-oxyde de N-méthylmorpholine (NMO) dans du dichlorométhane (DCM) (pour avoir une concentration du mélange de 0,35 mol/l), à température ambiante pendant 30 minutes. Le milieu réactionnel est filtré sur silice avec de l'éther puis évaporé. On obtient ainsi de la khusimone avec un rendement de 94%.

## Revendications

1. Procédé de synthèse de la khusimone par mise en présence du zizanal avec un réactif oxydant de type peracide ou peroxyde ou d'un mélange de peracides ou d'un mélange de peroxydes ou d'un mélange de peracides et de peroxydes eux-mêmes uniques ou en mélange.

2. Procédé selon la revendication 1, **caractérisé en ce que** le zizanal est mis en présence d'un réactif oxydant en présence d'une base.

3. Procédé selon la revendication 1, **caractérisé en ce que** le zizanal est mis en présence d'un réactif oxydant en présence d'un solvant organique.

4. Procédé selon la revendication 1, **caractérisé en ce que** le zizanal est mis en présence d'un réactif oxydant en présence d'une base et d'un solvant organique.

5. Procédé selon l'une quelconque des revendications 1 à4, **caractérisé en ce que** le dit réactif oxydant peut être choisi parmi l'eau oxygénée, l'hydroperoxyde de tert-butyle, le peroxyde de di-*tert*-butyle, le peroxyde de dicumyle, le peroxyde de *tert*-butyle et de cumyle, le peroxyde de dibenzoyle, de dilauryle, de di-(2.4-dichlorobenzoyle), l'hydroperoxyde de *tert*-butyle, de cumyle, de 1-phényléthyle, l'acide performique, l'acide peracétique, l'acide perpropionique, l'acide *m*-chloroperbenzoïque, l'acide monoperphtalique, l'acide monopermaléique ou encore l'acide trifluoroperacétique, avantageusement de l'eau oxygénée.

6. Procédé selon l'une quelconque des revendications 1 à 5, **caractérisé en ce que** le rapport molaire [(RO)/(Z)] entre le réactif oxydant (RO) et le zizanal (Z) est compris entre 0,1 et 50, préférentiellement entre 0,5 et 25, très préférentiellement entre 1 et 10, avantageusement égal à 6.

7. Procédé selon l'une quelconque des revendications 2, 4 à 6, **caractérisé en ce que** ladite base peut être choisie parmi la soude (NaOH), la potasse (KOH), le méthanolate de sodium (MeONa), l'hydroxyde de calcium (Ca(OH)₂), l'hydroxyde de lithium (LiOH), avantageusement de la potasse.

8. Procédé selon l'une quelconque des revendications 2, 4 à 7, **caractérisé en ce que** le rapport molaire [(B)/(Z)] entre la base (B) et le zizanal (Z) est compris entre 0,1 et 50, préférentiellement entre 0,5 et 25, très préférentiellement entre 1 et 10, avantageusement égale à 3.

9. Procédé selon l'une quelconque des revendications 3, 4 à 8, **caractérisé en ce que** ledit solvant est choisi parmi l'eau, le méthanol, l'éthanol, le propanol, l'isopropanol, le n-butanol, le *sec*-butanol, le *tert*-butanol, le tétrahydrofurane (THF), le 1,4-dioxane, le méthyl *tert*-butyl éther (MTBE), l'éther diéthylique (Et₂O), le glyme, le diglyme, le dichlorométhane (CH₂Cl₂), le chloroforme (CHCl₃), le diméthylsulfoxyde (DMSO), l'acétonitrile, l'acétate d'éthyle, l'acétate d'isopropyle ou des mélanges en toutes proportions de ces solvants, avantageusement le méthanol ou un mélange DMSO/méthanol dans des proportions 1/1.

10. Procédé selon l'une quelconque des revendications 1 à 9, **caractérisé en ce que** la concentration en zizanal dans le mélange réactionnel est comprise entre 0,01 et 4,5M, avantageusement entre 0,02 et 0,07M, très avantageusement 0,2M.

11. Procédé selon l'une quelconque des revendications 1 à 10, **caractérisé en ce que** la réaction est réalisée à température comprise entre -25°C et la température de reflux du solvant.

12. Procédé selon l'une quelconque des revendications 1 à 11, **caractérisé en ce qu'**il comprend en outre une étape d'oxydation du mélange 12-norzizaenol (IV) / khusimone (I) obtenu après la réaction de décarbonylation en milieu oxydant.

13. Procédé selon la revendication 12, **caractérisé en ce que** l'étape d'oxydation du mélange 12-norzizaenol (IV) / khusimone (I) est réalisée en présence d'un milieu oxydant (O) et d'un solvant organique.

14. Procédé selon la revendication 13, **caractérisé en ce que** le milieu oxydant (O) est choisi parmi le perruthénate de tétrapropylammonium, l'oxyde de manganèse, le pyridinum chlorochromate, le nitrate de cérium et d'ammonium (CAN),le pyridinium dichromate, l'oxydant de Collins, le trioxyde de chrome, le 2,3-dichloro-5,6-dicyano-p-benzoquinone (DDQ), la 2,4,6-trichloro-[1,3,5]-triazine, le DMSO,un mélange de DMSO et de chlorure d'oxalyle, un mélange de DMSO et de trioxyde de soufre pyridine, un mélange de DMSO et d'anhydride trifluoroacétique, un mélange de DMSO et d'anhydride acétique, un mélange de DMSO et d'oxyde de Phosphore (P₂O₅), un mélange de sulfure de diméthyle et de *N*-chlorosuccinimide (Me₂S/NCS oxydation de Corey-Kim), un mélange de H₂O₂ et de bicarbonate de potassium (KHCO₃) (oxydation de Fleming) le periodinane de Dess-Martin ou l'oxyde d'acétoxyiodine, l'acide 2-iodoxybenzoique, l'isopropoxyde d'aluminium, un système oxydant contenant le TEMPO (2,2,6,6-tetraméthylpiperidine-1-oxyl), ou le perruthénate de tetrapropylammonium (TPAP) et de la *N*-méthylmorpholine-*N-*oxyde, préférentiellement le perruthénate de tétrapropylammonium et éventuellement d'un co-oxydant tel que par exemple le N-oxyde de N-méthylmorpholine (NMO).

15. Procédé selon la revendication 13, **caractérisé en ce que** le solvant organique est choisi parmi le dichlorométhane (DCM), l'éther diéthylique, le DMSO, le toluène, le tétrahydrofurane (THF), ou encore l'acétone, l'acétonitrile, l'eau, ou un mélange en toutes proportions des solvants cités, préférentiellement le DCM, l'acétone, le DMSO et l'eau ou un mélange en toutes proportions des solvants cités.

## Patentansprüche

1. Verfahren zum Synthetisieren von Khusimon durch Inkontaktbringen von Zizanal mit einem oxidierenden Reagens des Persäure- oder Peroxidtyps oder eines Persäuregemischs oder eines Peroxidgemischs oder eines Gemischs von Persäuren und Peroxiden, die wiederum allein oder im Gemisch sind.

2. Verfahren nach Anspruch 1, **dadurch gekennzeichnet, dass** das Zizanal in Anwesenheit einer Base mit einem oxidierenden Reagens in Kontakt gebracht wird.

3. Verfahren nach Anspruch 1, **dadurch gekennzeichnet, dass** das Zizanal in Anwesenheit eines organischen Lösungsmittels mit einem oxidierenden Reagens in Kontakt gebracht wird.

4. Verfahren nach Anspruch 1, **dadurch gekennzeichnet, dass** das Zizanal in Anwesenheit einer Base und eines organischen Lösungsmittels mit einem oxidierenden Reagens in Kontakt gebracht wird.

5. Verfahren nach einem der Ansprüche 1 bis 4, **dadurch gekennzeichnet, dass** das genannte oxidierende Reagens ausgewählt werden kann aus Wasserstoffperoxid, *tert*-Butyl-hydroperoxid, Di-*tert*-butyl-peroxid, Dicumylperoxid, *tert*-Butyl- und Cumyl-Peroxid, Dibenzoyl-, Dilauryl-, Di-(2,4-dichlorbenzoyl)-peroxid, *tert-Butyl-,* Cumyl-, 1-phenylethyl-hydroperoxid, Perameisensäure, Peressigsäure, Perpropionsäure, *m*-Chlorperbenzoesäure, Monoperphthalsäure, Monopermaleinsäure oder Trifluorperessigsäure, vorteilhafterweise Wasserstoffperoxid.

6. Verfahren nach einem der Ansprüche 1 bis 5, **dadurch gekennzeichnet, dass** das Molverhältnis [(RO)/(Z)] zwischen dem oxidierenden Reagens (RO) und dem Zizanal (Z) zwischen 0,1 und 50, vorzugsweise zwischen 0,5 und 25, sehr bevorzugt zwischen 1 und 10, vorteilhafterweise bei gleich 6 liegt.

7. Verfahren nach einem der Ansprüche 2, 4 bis 6, **dadurch gekennzeichnet, dass** die genannte Base ausgewählt werden kann aus Soda (NaOH), Kalium (KOH), Natriummethanolat (MeONa), Calciumhydroxid (Ca(OH)₂), Lithiumhydroxyd (LiOH), vorteilhafterweise Kalium.

8. Verfahren nach einem der Ansprüche 2, 4 bis 7, **dadurch gekennzeichnet, dass** das Molverhältnis [(B)/(Z)] zwischen der Base (B) und dem Zizanal (Z) zwischen 0,2 und 50, vorzugsweise zwischen 0,5 und 25, sehr bevorzugt zwischen 1 und 10, vorteilhafterweise bei gleich 3 liegt.

9. Verfahren nach einem der Ansprüche 3, 4 bis 8, **dadurch gekennzeichnet, dass** das genannte Lösungsmittel ausgewählt wird aus Wasser, Methanol, Ethanol, Propanol, Isopropanol, n-Butanol, sec-Butanol, *tert*-Butanol, Tetrahydrofuran (THF), 1,4-Dioxan, Methyl-*tert*-Butylether (MTBE), Diethylether (Et₂O), Glyme, Diglyme, Dichlormethan (CH₂Cl₂), Chloroform (CHCl₃), Dimethylsulfoxid (DMSO), Acetonitril, Ethylacetat, Isopropylacetat oder Mischungen in allen Anteilen dieser Lösungsmittel, vorteilhafterweise Methanol oder ein DMSO/Methanol-Gemisch im Verhältnis 1:1.

10. Verfahren nach einem der Ansprüche 1 bis 9, **dadurch gekennzeichnet, dass** die Zizanal-Konzentration im Reaktionsgemisch zwischen 0,01 und 4,5 M, vorteilhafterweise zwischen 0,02 und 0,07 M, sehr vorteilhafterweise bei 0,2 M liegt.

11. Verfahren nach einem der Ansprüche 1 bis 10, **dadurch gekennzeichnet, dass** die Reaktion bei einer Temperatur zwischen -25°C und der Rückflusstemperatur des Lösungsmittels erfolgt.

12. Verfahren nach einem der Ansprüche 1 bis 11, **dadurch gekennzeichnet, dass** es ferner einen Schritt des Oxidierens des Gemischs 12-Norzizaenol (IV) / Khusimon (I) beinhaltet, erhalten nach der Decarbonylierungsreaktion in dem oxidierenden Medium.

13. Verfahren nach Anspruch 12, **dadurch gekennzeichnet, dass** der Schritt des Oxidierens des Gemischs 12-Norzizaenol (IV) / Khusimon (I) in Anwesenheit eines oxidierenden Mediums (O) und eines organischen Lösungsmittels erfolgt.

14. Verfahren nach Anspruch 13, **dadurch gekennzeichnet, dass** das oxidierende Medium (O) ausgewählt wird aus Tetrapropylammoniumperruthenat, Manganoxid, Chlorchromatpyridinium, Cerium- und Ammoniumhydrat (CAN), Pyridiniumdichromat, Collins-Oxidationsmittel, Chromtrioxid, 2,3-Dichlor-5,6-dicyano-p-benzochinon (DDQ), 2,4,6-Trichlor[1,3,5]-triazin, DMSO, einem Gemisch aus DMSO und Oxalylchlorid, einem Gemisch aus DMSO und Pyridinschwefeltrioxid, einem Gemisch aus DMSO und Trifluoressigsäureanhydrid, einem Gemisch aus DMSO und Essigsäureanhydrid, einem Gemisch aus DMSO und Phosphoroxid (P₂O₅), einem Gemisch aus Dimethylsulfid und *N*-Chlorsuccinimid (Me₂S/NCS-Oxidation von Corey-Kim), einem Gemisch aus H₂O₂ und Kaliumbicarbonat (KHCO₃) (Fleming-Oxidation), Dess-Martin-Periodinan oder Acetoxyiodoxid, 2-lodoxybenzoesäure, Aluminiumisopropoxid, einem Oxidationssystem, das TEMPO (2,2,6,6-Tetramethylpiperidin-1-oxyl) enthält, oder Tetrapropylammoniumperruthenat (TPAP) und *N*-Methylmorpholin-*N*-oxid, vorzugsweise Tetrapropylammoniumperruthenat und eventuell einem Cooxidationsmittel wie zum Beispiel das N-Oxid von N-Methylmorpholin (NMO).

15. Verfahren nach Anspruch 13, **dadurch gekennzeichnet, dass** das organische Lösungsmittel ausgewählt wird aus Dichlormethan (DCM), Diethylether, DMSO, Toluol, Tetrahydrofuran (THF) oder Aceton, Acetonitril, Wasser oder einem Gemisch in allen Anteilen der erwähnten Lösungsmittel, vorzugsweise DCM, Aceton, DSMO und Wasser oder einem Gemisch in allen Anteilen der erwähnten Lösungsmittel.

## Claims

1. Method for the synthesis of khusimone by bringing zizanal into contact with an oxidizing reagent of the peracid or peroxide type or a mixture of peracids or a mixture of peroxides or a mixture of peracids and peroxides which are themselves alone or in a mixture.

2. Method according to claim 1, **characterized in that** zizanal is brought into contact with an oxidizing reagent in the presence of a base.

3. Method according to claim 1, **characterized in that** zizanal is brought into contact with an oxidizing reagent in the presence of an organic solvent.

4. Method according to claim 1, **characterized in that** zizanal is brought into contact with an oxidizing reagent in the presence of a base and an organic solvent.

5. Method according to any one of claims 1 to 4, **characterized in that** said oxidizing reagent can be selected from hydrogen peroxide, *tert*-butyl hydroperoxide, di-*tert*-butyl peroxide, dicumyl peroxide, *tert*-butyl cumyl peroxide, dibenzoyl peroxide, dilauryl, di-(2,4-dichlorobenzoyl), *tert*-butyl hydroperoxide, cumyl, 1-phenylethyl, performic acid, peracetic acid, perpropionic acid, *m*-chloroperbenzoic acid, monoperphthalic acid, monopermaleic acid or also trifluoroperacetic acid, advantageously hydrogen peroxide.

6. Method according to any one of claims 1 to 5, **characterized in that** the molar ratio [(OR)/(Z)] between the oxidizing reagent (OR) and the zizanal (Z) is comprised between 0.1 and 50, preferentially between 0.5 and 25, very preferentially between 1 and 10, advantageously equal to 6.

7. Method according to any one of claims 2, 4 to 6, **characterized in that** said base can be selected from sodium hydroxide (NaOH), potassium hydroxide (KOH), sodium methoxide (MeONa), calcium hydroxide (Ca(OH)₂), lithium hydroxide (LiOH), advantageously potassium hydroxide.

8. Method according to any one of claims 2, 4 to 7, **characterized in that** the molar ratio [(B)/(Z)] between the base (B) and the zizanal (Z) is comprised between 0.1 and 50, preferentially between 0.5 and 25, very preferentially between 1 and 10, advantageously equal to 3.

9. Method according to any one of claims 3, 4 to 8, **characterized in that** said solvent in selected from water, methanol, ethanol, propanol, isopropanol, *n*-butanol, *sec-*butanol, *tert*-butanol, tetrahydrofuran (THF), 1,4-dioxane, methyl *tert*-butyl ether (MTBE), diethyl ether (Et₂O), glyme, diglyme, dichloromethane (CH₂Cl₂), chloroform (CHCl₃), dimethyl sulphoxide (DMSO), acetonitrile, ethyl acetate, isopropyl acetate or mixtures in any proportions of these solvents, advantageously methanol or a DMSO/methanol mixture in proportions of 1/1.

10. Method according to any one of claims 1 to 9, **characterized in that** the concentration of zizanal in the reaction mixture is comprised between 0.01 and 4.5M, advantageously between 0.02 and 0.07M, very advantageously 0.2M.

11. Method according to any one of claims 1 to 10, **characterized in that** the reaction is carried out at a temperature comprised between -25°C and the reflux temperature of the solvent.

12. Method according to any one of claims 1 to 11, **characterized in that** it also comprises a step of oxidizing the 12-norzizaenol (IV) / khusimone (I) mixture obtained after the decarbonylation reaction in an oxidizing medium.

13. Method according to claim 12, **characterized in that** the step of oxidizing the 12-norzizaenol (IV) / khusimone (I) mixture is carried out in the presence of an oxidizing medium (O) and an organic solvent.

14. Method according to claim 13, **characterized in that** the oxidizing medium (O) is selected from tetrapropylammonium perruthenate, manganese oxide, pyridinium chlorochromate, ceric ammonium nitrate (CAN), pyridinium dichromate, Collins reagent, chromium trioxide, 2,3-dichloro-5,6-dicyano-p-benzoquinone (DDQ), 2,4,6-trichloro-[1,3,5]-triazine, DMSO, a mixture of DMSO and oxalyl chloride, a mixture of DMSO and sulphur trioxide pyridine, a mixture of DMSO and trifluoroacetic anhydride, a mixture of DMSO and acetic anhydride, a mixture of DMSO and phosphorus oxide (P₂O₅), a mixture of dimethyl sulphide and *N*-chlorosuccinimide (Me₂S/NCS Corey-Kim oxidation), a mixture of H₂O₂ and potassium bicarbonate (KHCO₃) (Fleming oxidation), Dess-Martin periodinane or acetoxyiodine oxide, 2-iodoxybenzoic acid, aluminium isopropoxide, an oxidizing system containing TEMPO (2,2,6,6-tetramethylpiperidine-1-oxyl) or tetrapropylammonium perruthenate (TPAP) and *N*-methylmorpholine-*N*-oxide, preferentially tetrapropylammonium perruthenate and optionally a co-oxidant such as for example N-methylmorpholine N-oxide (NMO).

15. Method according to claim 13, **characterized in that** the organic solvent is selected from dichloromethane (DCM), diethyl ether, DMSO, toluene, tetrahydrofuran (THF), or also acetone, acetonitrile, water or a mixture in any proportions of the above-mentioned solvents, preferentially DCM, acetone, DMSO and water or a mixture in any proportions of the above-mentioned solvents.
